# EUROPEAN PATENT SPECIFICATION

(11) **EP 0 539 522 B1**
(45) Date of publication and mention of the grant of the patent: **30.12.1998**
(21) Application number: 91915687.7
(22) Date of filing: 19.07.1991
(51) Int. Cl.: A61K 39/42

(54) **IMPROVED IMMUNOTHERAPEUTIC METHOD OF PREVENTING OR TREATING VIRAL RESPIRATORY TRACT DISEASE**
VERBESSERTE IMMUNOTHERAPEUTISCHE METHODE ZUR VERHINDERUNG ODER BEHANDLUNG VIRALER KRANKHEITEN DER ATEMWEGE
PROCEDE IMMUNOTHERAPEUTIQUE AMELIORE POUR LA PREVENTION OU LE TRAITEMENT DES MALADIES VIRALES DES VOIES RESPIRATOIRES

(30) Priority: 19.07.1990 US 555091
(43) Date of publication of application: 05.05.1993
(73) Proprietor: THE UNITED STATES OF AMERICA as represented by the Secretary United States Department of Commerce, Springfield, Virginia 22161 (US)
(72) Inventor: CHANOCK, Robert, Merritt, Bethesda, MD 20817 (US); PRINCE, Gregory, Antone, Potomac, MD 20854 (US); COELINGH, Kathleen, Louise, San Francisco, CA 94114 (US); MURPHY, Brian, Robert, Bethesda, MD 20816 (US); HEMMING, Val, Gaithersburg, MD 20879 (US); BEELER, Judy, Allyson, Bethesda, MD 20814 (US)
(74) Representative: Pett, Christopher Phineas
(86) International application number: US9105002
(87) International publication number: WO9201473

(56) References cited:
- WO-A-91/16074
- WO-A-92/04381
- US-A- 4 800 078
- US-A- 4 933 169
- US-A- 4 965 069
- JOURNAL OF VIROLOGY vol. 63, no. 7, July 1989, BALTIMORE, PA, US pages 2941 - 2950 J.A. BEELER ET AL. 'NEUTRALIZATION EPITOPES OF THE F GLYCOPROTEIN OF RESPIRATORY SYNCYTIAL VIRUS: EFFECT OF MUTATION UPON FUSION FUNCTION.'
- CLINICAL CHEMISTRY vol. 35, no. 9, 1989, WINSTON-SALEM, N.C., US pages 1849 - 1853 G.P. MOORE 'GENETICALLY ENGINEERED ANTIBODIES.'
- JOURNAL OF VIROLOGY vol. 64, no. 6, June 1990, BALTIMORE, PA, US pages 3091 - 3092 G.A. PRINCE ET AL. 'MECHANISM OF ANTIBODY-MEDIATED VIRAL CLEARANCE IN IMMUNOTHERAPY OF RESPIRATORY SYNCYTIAL VIRUS INFECTION OF COTTON RATS.'
- VACCINES 91: MODERN APPROACHES TO NEW VACCINES INCLUDING PREVENTION OF AIDS. ( 8TH ANNUAL MEETING, COLD SPRING HARBOR, NEW YORK, US, SEPTEMBER 1990) vol. 91, 1991, NEW YORK, N.Y., US pages 283 - 288 G.P. BANSAL ET AL. 'EFFICACY OF PASSIVELY ADMINISTERED MONOCLONAL ANTIBODIES AGAINST RESPIRATORY SYNCYTIAL VIRUS INFECTION IN COTTON RATS.'
- Journal of Virology, Volume 63, Number 1, issued January 1989, COELINGH et al.,
- "Antigenic and Functional organization of Human Para influensa Virus Type 3 Fusion Glycoprotein", pages 375-382, see the entire document.
- Journal of Virology, Volume 62, Number 11, issued November 1988, ANDERSON et al., "Neutralization of Respiratory Syncytial Virus by Individual and Mixtures of F and G protein monoclonal antibodies", pages 4232-4238, see the entire document.

## Description

The present invention is related generally to immunotherapy of respiratory disease caused by viral infection. More particularly, the present invention is related to an improved immunological method of preventing or treating viral respiratory tract disease by administering into the lower respiratory tract of a susceptible host a small particle (<2µm) aerosol of a mixture of specific monoclonal antibodies directed against the various protective antigenic sites present on a major protective viral surface antigen (or antigens).

Anderson *et al*., J. Virology 62(11), 4232-4238 (1988) refers generally to the study of the mechanisms of protective immunity, i.e. the eliciting of antibodies against respiratory syncytial virus (RSV). It does not address immunotherapy with monoclonal antibodies or describe neutralisation assays in intact hosts.

WO91/16074 (state of the art under Art. 54(3) EPC) refers to a process for the prophylaxis and/or treatment of respiratory viruses which comprises administering topically or systemically, to an animal, at least one monoclonal antibody against at least one respiratory virus, in particular, against respiratory syncytial virus (RSV). No mention of aerosol administration of monoclonal antibodies to the lower respiratory tract is made.

An immunotherapeutic method of treating pulmonary disease due to respiratory syncytial virus (RSV) by intranasal administration of a purified human immunoglobulin preparation (designated IVIG because it is prepared as a monomeric suspension of human IgG that is suitable for administration by the intravenous route) containing a high titer of neutralizing antibodies (^{∼}1:1000 to 1:2000) has been described in U.S. patent 4,800,078. In this instance IVIG was administered into the nose of anesthetized cotton rats which resulted in quantitative delivery of IVIG into the lungs, thereby simulating the aerosol mode of administration that is used in humans for pulmonary therapy. Also described in this patent was the important discovery that topically administered human immunoglobulins (IVIG) with a high titer of RSV neutralizing antibodies were retained in the lungs for up to 7 days and provided a significant prophylactic effect against RSV infection throughout this interval. However, with the presently available technology, direct introduction of the requisite amount of these antibodies into the lungs of infants and young children, who are most susceptible to RSV or other respiratory viral disease, can be achieved effectively only for those patients who are intubated in order to be connected to a mechanical ventilator which assists the respiratory function of the patient. It is clear that it would be difficult as well as unsafe to deliver the requisite amount of an IVIG suspension of 24 mg per kg, i.e., 125 mg to 250 mg of human immunoglobulin IgG for a 5 to 10 kg infant or young child, into the lungs of a patient who was not intubated. An improved therapeutic modality is, therefore, needed.

### SUMMARY OF THE INVENTION

It is, therefore, an object of the present invention to provide the use of a pooled suspension of different, purified monoclonal antibodies or F(ab')₂ fragments thereof, directed against conserved epitopes located on major protective antigenic sites of protective antigens of a disease causing virus in tile preparation of a small particle aerosol adapted to dispense particles of size equal to or less than about 2 µm for the treatment or prevention of viral respiratory tract disease.

Pursuant to this goal it is a further object of the present invention to utilize a mixture of neutralizing and/or therapeutic and/or prophylactic monoclonal antibodies, each of which has a different antigenic specificity that is directed to a specific highly conserved epitope on the surface of a major protective viral protein. Also, these epitopes against which the monoclonal antibodies are directed collectively represent the known repertoire of the major protective viral surface antigenic sites, i.e., monoclonal antibody to at least one epitope in each major protective antigenic site is included in the monoclonal antibody mixture. The mixture contains monoclonal antibodies suitable for human use and directed to highly conserved epitopes that are: (1) shared by the known viral antigenic subgroups, such as RSV subgroup A and subgroup B, or (ii) unique to a viral antigenic subgroup, such as RSV subgroup A or subgroup B. This strategy is dictated by the need to: (i) protect against infection of (ii) treat infection by a large variety or virus strains that exhibit antigenic variation in some of the protective antigenic sites on viral surface proteins.

Other objects and advantages will become evident from the following detailed description of the invention.

### BRIEF DESCRIPTION OF THE DRAWINGS

These and other objects, features, and many of the attendant advantages of the invention will be better understood upon a reading of the following detailed description when considered in connection with the accompanying drawings wherein:

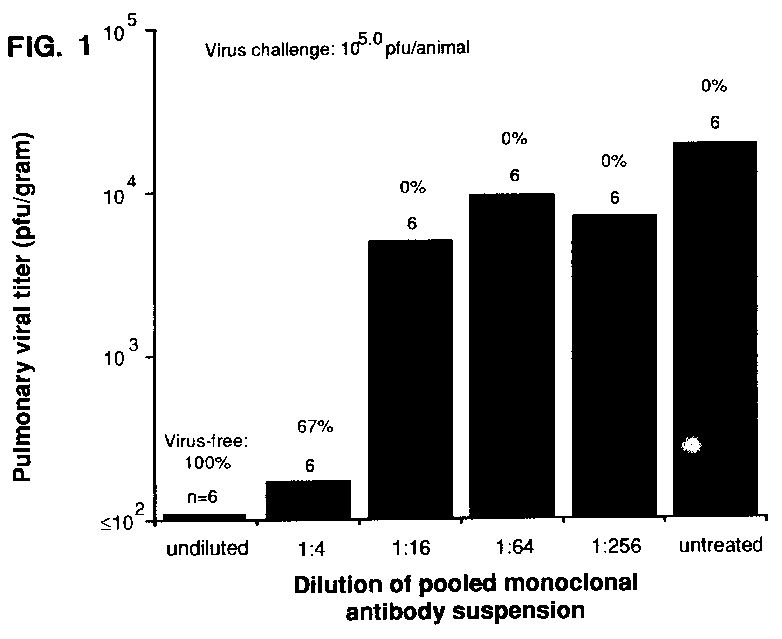

Figure 1 shows the therapeutic efficacy of a mixture of 16 neutralizing monoclonal antibodies directed against antigenic sites A, AB, B, or C of the RSV fusion (F) surface glycoprotein with neutralizing antibody titers ranging from 1:320 to 1:5120, and one monoclonal antibody without neutralizing activity, directed against site B (Beeler et al, J. Virol, 63:2941-2950, 1989). The RSV F monoclonal antibodies were combined to form an antibody mixture in which the geometric mean titer of the neutralizing antibodies was 1:1100. 0.1 ml of the antibody suspension was administered by the intranasal route to anesthetized cotton rats (average weight 100 grams) 3 days after infection with RSV, and the quantity of the RSV present in the lungs was titrated 24 hours later.

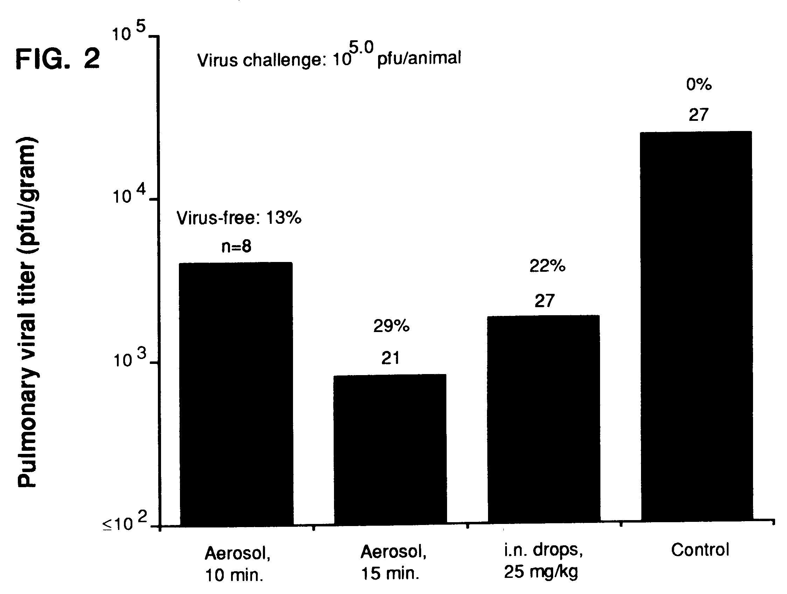

Figure 2 shows the results of aerosol immunotherapy with RSV neutralizing antibodies. Three days after infection with RSV a 5% suspension of purified pooled human immunoglobulin G (IVIG), with a RSV neutralizing antibody titer of 1:800, was administered by: (i) small particle (<2µm) aerosol or (ii) intranasal drops (25 mg/kg) to anesthetized cotton rats (average weight 100 grams) and the lungs were titrated for RSV 24 hours later.

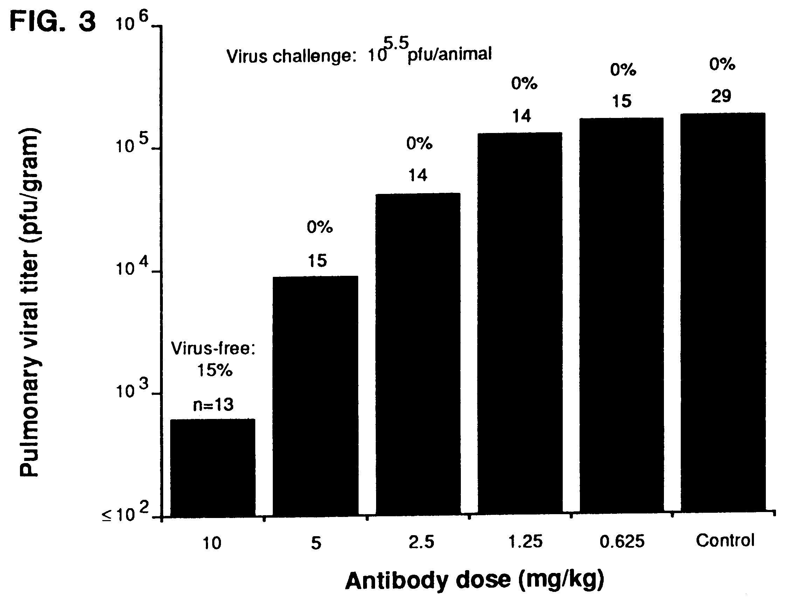

Figure 3 shows the effectiveness of topical immunotherapy of human parainfluenza virus type 3 (HPIV3). A pool of cotton rat human parainfluenza type 3 virus (HPIV3) convalescent sera, with an HPIV3 neutralizing antibody titer of 1:10,000, was administered by the intranasal route to anesthetized cotton rats (average weight 100 grams) 3 days after infection with HPIV3 and the lungs were titrated for the amount of virus present 24 hours later.

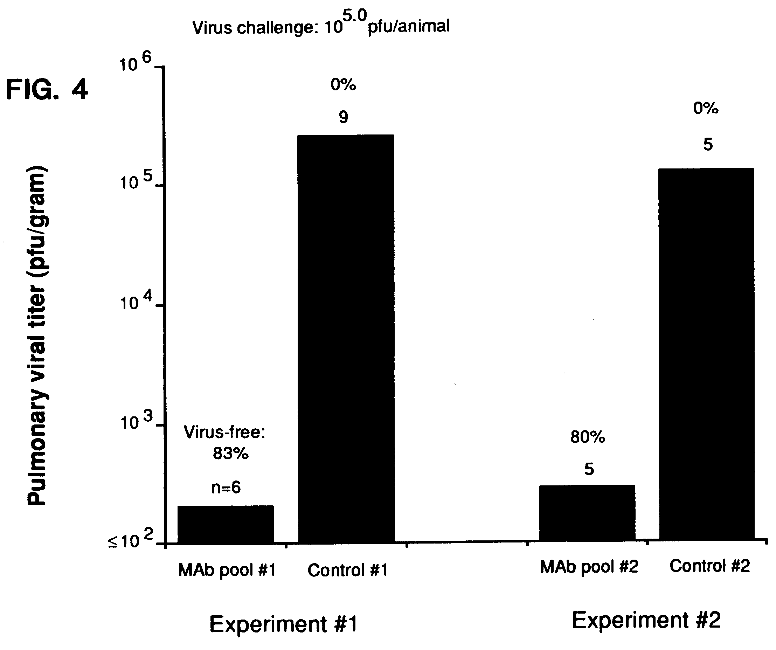

Figure 4 shows the effectiveness of a mixture of neutralizing HPIV3 monoclonal antibodies in systemic passive immunoprophylaxis of HPIV3 infection. Monoclonal antibody (MAb) pool #1 contained 4 neutralizing monoclonal antibodies directed against antigenic site A or the viral surface hemagglutinin-neuraminidase (HN) glycoprotein of HPIV3 and 1 neutralizing monoclonal antibody directed against site B (Coelingh et al, Virology 143:;569-582, 1985). MAb pool #2 contained the aforementioned 5 MAbs plus an additional neutralizing MAb directed against antigen site A of the HPIV3 HN. Infant cotton rats (average weight 10 grams) were inoculated intraperitoneally with 0.4 ml of the mixture of monoclonal antibodies (derived from ascites fluid) per 10 grams body weight. 24 hours later anesthetized infants were challenged intranasally with HPIV3, and the lungs were titrated for the amount of virus present 4 days later. The mean HPIV3 serum neutralizing antibody titer of cotton rats at the time of virus challenge was 1:6400 in Experiment #1 and 1:3200 in Experiment #2.

### DETAILED DESCRIPTION OF THE INVENTION

The above and various other objects and advantages of the present invention are achieved by the use of a pooled suspension of different, purified monoclonal antibodies or F(ab')₂ fragments thereof, directed against conserved epitopes located on major protective antigenic sites of protective antigens of a disease causing virus in the preparation of a small particle aerosol adapted to dispense particles of size equal to or less than about 2 µm for the treatment or prevention of viral respiratory tract disease.

It should be noted that there are several factors leading to the evolution of the inventive methoaology described herein. First, the use of human immunoglobulin IgG suitable for administration by the intravenous route (IVIG) described in previous reports requires a preparation of purified, monodispersed IgG derived from a pool of human plasma. This is problematic from the point of view of available amounts of human IgG with high titer of virus neutralizing antibodies. There is also a possible risk of infectious contaminants and the like which may be present in the plasma. However, it was found that the combination of the appropriate monoclonal antibodies provided an inexhaustible supply of efficacious reagents for the prophylactic and therapeutic usage described herein. Also, the use of monoclonal antibodies decreases the amount of immunoglobulin suspension required for a topical therapeutic effect by a factor of 10 to 50 or more. RSV or HPIV3 antibodies (or other viral antibodies) constitute only a very small proportion of the immunoglobulin molecules in murine or human immunoglobulin IgG. In contrast. a suspension of (i) purified murine monoclonal antibodies, (ii) purified human neutralizing monoclonal antibodies derived from lambda phage recombinant-infected E. coli expressing the Fab fragment of such antibodies (as described recently by Huse et al., Science 246:1275-1281, 1989) or (iii) purified "humanized" mouse monoclonal antibodies produced by recombinant DNA technology to contain the variable region antigen combining sites of murine monoclonal antibodies with the remaining regions of the molecules being of human immunoglobulin origin (as described by Reichman et al., Nature 332:323-327, 1988), would contain essentially only the relevant virus neutralizing and/or prophylactic and/or therapeutic antibodies. This would decrease the dose of IgG required for a therapeutic effect by a factor of 10 to 50 or more. Second, the therapeutic efficacy of the RSV neutralizing antibodies delivered directly into the lungs in a small particle aerosol (<2.0µm), which is specifically targeted to the smallest air passages and alveoli, has been demonstrated in cotton rats and constitutes an important feature of the current invention. In a similar manner 2.5 to 25 mg of RSV neutralizing antibodies could be delivered directly into the lungs of young patients by aerosol during a short interval and the therapeutic effect of these antibodies would be experienced soon after administration. This represents a major advantage of topical therapy in addition to its greater efficiency compared to parenterally administered antibodies. For example, in infants and young children with severe RSV lower respiratory tract disease the time required to safely attain a therapeutic level of serum RSV neutralizing antibodies by intravenous administration of purified human immunoglobulin IgG (IVIG) ranges from 2 to 4 hours. It should be noted that parenterally administered antibodies exert their therapeutic effect in the lungs following transudation onto the lumenal mucosal or epithelial surface of the lungs and the concentration of such antibodies on the lumenal surface appears to be at least two orders of magnitude less than in serum. In contrast, a therapeutic level of these antibodies in the lungs can be attained with considerably greater efficiency (^{∼}100 times greater efficiency) within a few minutes following aerosol administration of: (i) immunoglobulin IgG (IVIG) with very high RSV, HPIV3 or other viral neutralizing activity or (ii) a purified suspension of neutralizing and/or protective and/or therapeutic monoclonal antibodies with even higher specific activity which would decrease the amount of immunoglobulin required for therapy of prophylaxis to a level which could be delivered by current aerosol technology to unintubated patients.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. Unless mentioned otherwise, the techniques employed or contemplated herein are standard methodologies well known to one or ordinary skill in the art. The materials, methods and examples are illustrative only and not limiting.

### MATERIALS AND METHODS

### RSV Monoclonal Antibodies

Murine monoclonal antibodies directed against 16 distinct epitopes present on the 4 known neutralization antigenic sites of the F glycoprotein of RSV and conserved among subgroup A and subgroup B strains of RSV were prepared by standard methodologies well known to one of ordinary skill in the art and pooled for treating cotton rats previously infected with RSV. Cotton rats were inoculated intranasally with 10⁵ plaque-forming units (PFU) of RSV. Three days later, at the peak of viral replication, the animals were anesthetized and inoculated intranasally with 0.1 ml of a dilution of the pooled monoclonal antibody suspension. One day after treatment the animals were sacrificed, and pulmonary viral titers determined by plaque assay.

As shown in Fig. 1, at a 1:4 dilution (i.e., an antibody suspension with a neutralizing titer of 1:275) the pooled monoclonal antibody preparation of the present invention effected a 100-fold reduction in pulmonary viral titer. It should be noted that while the percentage of antibody molecules in human plasma directed against RSV is small (about 0.1% or less), purified "humanized" (murine-human recombinant monoclonal antibodies) or purified human monoclonal antibodies derived from Fab fragments of such antibodies produced in E. coli, by definition, have 100% specificity. Thus, a preparation of pooled monoclonal antibodies which can be prepared by hybridoma and recombinant DNA techniques (Riechmann et al., Nature 332:323-327, 1988) or by direct cloning and expression of Fab fragments in E. coli using recombinant DNA technology (Huse et al., Science 246:1275-1281, 1989) and which have activity against both A and B subgroups or RSV should yield a suspension as much as 50-fold or more potent than purified IgG (IVIG) derived from human plasma.

It may be noted that a mixture of several MAbs directed against different antigenic sites of the viral protein(s) would be required for maximal therapeutic or prophylactic efficacy. The use of a single MAb should not be as effective as a combination of MAbs directed against different antigenic sites because of the high frequency of mutants that acquire resistance to neutralization by a specific monoclonal antibody directed against a single epitope. The frequency of such a "neutralization escape mutation" affecting a single epitope is ^{∼}1 mutant for every 10⁴ to 10⁵ virus particles produced during virus replication. Hence, the use of monoclonal antibodies representing different antigenic sites reduces the frequency of neutralization escape mutants as a function of the product of the frequency of such mutants for each site represented in the monoclonal antibody mixture. For example, when monoclonal antibodies for 2 antigenic sites are used in prophylaxis or therapy the frequency of "neutralization escape mutants" would be 10⁻⁴ to 10⁻⁵ X 10⁻⁴ to 10⁻⁵(i.e., 10⁻⁸ to 10¹⁰), for 3 antigenic sites 10⁻¹² to 10⁻¹⁶ and for 4 antigenic sites 10⁻¹⁶ to 10⁻²⁰.

F(ab)₂ fragments of human or murine neutralizing antibodies can also be used for topical therapy of RSV or other respiratory viral infection. Recent studies summarized in Table 1 demonstrate that F(ab)₂ fragments of human RSV neutralizing antibodies are as effective in topical therapy of RSV infection of cotton rats as the full immunoglobulin molecules from which the F(ab)₂ fragments were derived (Prince et al., J. Virol. In press 1990). Cotton rats were infected intranasally with 10^{5.0} PFU of RSV (strain A2) on day 0, anesthetized animals were treated with topical IgG or F(ab')₂ by direct instillation into the respiratory tracts on day 3, and the animals were sacrificed on day 4 and the lungs were titrated for the quantity of RSV present; days 3 and 4 represent the time of peak viral titer in the lungs. Concurrently infected animals which were not treated with IgG of F(ab')₂ served as the basis for determining the extent of viral clearance effected by topical immunotherapy. When used at an equimolar concentration, IgG (group 2) and F(ab')₂ (group 5) were equally effective, each bringing about a 100-fold reduction in pulmonary virus. The possibility that the small amount of Fc remaining in the F(ab')₂ preparation (<0.2%) contributed to viral clearance was examined by using 10-fold (group 3) and 100-fold (group 4) dilutions of IgG. The IgG administered to group 4 contained a 10-fold higher amount of Fc than that found in the F(ab')₂ preparation but had no effect on viral titer, indicating that the antiviral effect seen in group 5 was due exclusively to F(ab')₂. The role of complement was studied by comparing the efficacy of antibody in clearing virus from complement-depleted and-sufficient animals (Table 1). Cobra venom factor (Naja Naja; Cordis Laboratories, Inc., Miami, Fla.) was administered intracardially, at a dose of 200 U per kg of body weight, to cotton rats challenged 2 days earlier with 10^{5.0} PFU of RSV. These animals (group 7) were treated with topically administered IgG 24 h after administration of Cobra venom factor (day 3). On day 4 the animals were sacrificed, and viral titers in lung homogenates were determined. Serum 50% hemolytic complement titers at the time of IgG administration (guinea pig C test kit, catalog No. 737-303; Cordis) were greater than 1:5,600 in complementsufficient animals, whereas a 1:50 dilution of serum of Cobra venom factor-treated animals did not contain detectable complement activity. Complement-depleted animals treated with IgG (group 7) showed a greater than 100-fold decrease in virus titer compared with the untreated, complement-sufficient (group 1) and untreated, complemented-depleted (group 6) animals. Complement-sufficient animals treated with IgG (group 2) showed a similar level of viral clearance as that seen in group 7, indicating that antibody-mediated clearance of RSV is not dependent on complement. Additionally, complement-depleted cotton rats were treated with F(ab')₂ (group 8) and were found to experience a similar degree of viral clearance as that in groups 2, 5 and 7.

### Comparison of Efficacy of Aerosol and Dropwise Intranasal Administration of IgG

A small-particle ultrasonic nebulizer (Portasonic 8500 D. DeVilbiss Co., Somerset, PA) was used to generate aerosol particles (<2 µm) and to deliver the antibodies into the lungs of anesthetized cotton rats (Fig. 2). Optimal results were obtained using a 5% suspension of purified human IgG (IVIG) because more concentrated suspensions became frothy when used for production of an aerosol. Animals were inoculated intranasally with 10⁵ PFU of RSV. Three days later they were anesthetized and administered IVIG by: (i) small particle (<2.0 µm) aerosol during a 10 or 15 minute exposure, or (ii) by intranasal drops (Fig. 2). In the former instance, cotton rats were allowed to breath an IgG aerosol as it emerged from the exit orifice of the chamber lid of a Porta-Sonic Nebulizer Model 8500D (DeVilbiss). A 15-minute exposure to aerosolized IgG effected a 30-fold reduction in pulmonary virus which was slightly more effective than a dose of 25 mg IgG per kg delivered dropwise into the respiratory tract of anesthetized cotton rats.

The results obtained are important for two reasons: (1) They show that IgG directed against RSV can be delivered topically with an aerosol nebulizer already in widespread clinical use. (2) The exposure time required for significant reduction in pulmonary virus (i.e., 15 minutes) compares very favorably with ribavirin, the only antiviral drug currently approved for treatment of RSV disease in human infants, which requires daily aerosol treatment of up to 18 hours for 3 to 5 days.

### Parainfluenza Virus

a) Treatment. Preparations of purified human IgG (IVIG) which were effective in treating RSV infection in cotton rats had a neutralizing antibody titer against RSV approximately 50-fold higher than against human parainfluenza virus type 3 (HPIV3). Whereas these preparations effected a 100-fold or greater reduction in pulmonary titer or RSV in infected cotton rats, the same preparations reduced pulmonary HPIV3 titers only 3-fold (data not shown). Therefore, a pool of HPIV3 convalescent cotton rat serum with a neutralizing antibody titer against HPIV3 of 1:10,000 was prepared by standard methodology well known to one of ordinary skill in the art and used for investigating the feasibility of topical immunotherapy of HPIV3 (Fig. 3).
   Cotton rats were inoculated intranasally with 10^{5.5} PFU of HPIV3. Three days later anesthetized animals were treated intranasally with 0.1 ml of a dilution of the convalescent cotton rat serum pool (Fig. 3). One day after treatment the animals were sacrificed and pulmonary viral titers determined.
   An HPIV3 convalescent serum dose of 10 mg/kg effected a 100-fold reduction in pulmonary virus titer. The fact that the amount of neutralizing antibodies required for a 100-fold reduction in titer of HPIV3 was several times higher than required for RSV, probably reflects the greater permissiveness of cotton rats for HPIV3 infection. HPIV3 replicates in the cotton rat lung to a titer 10- to 50-fold higher than RSV.
   Therapeutic efficacy of HPIV3 antibodies in this experiment demonstrates the effectiveness of topical immunotherapy for another important human viral respiratory pathogen and clearly indicates that (i) the usefulness of topical immunotherapy is not limited to RSV infection and (ii) topical immunotherapy should prove useful for other respiratory viral pathogens, such as parainfluenza virus types 1, 2 and 4, and influenza A and influenza B viruses whose pathogenic effects are also limited to the cells which line the lumen of the respiratory tract.
b) Prophylaxis. Monoclonal antibodies directed against the A and B neutralization sites on the HN glycoprotein of HPIV3 were pooled and administered intraperitoneally to infant cotton rats. One day later the animals were challenged intranasally with 10^{5.0} PFU of HPIV3. Four days thereafter the animals were sacrificed and pulmonary viral titers determined (Fig. 4). At the time of challenge, the HPIV3 neutralizing antibody titer in the serum of monoclonal antibody recipients ranged from 1:3200 to 1:6400. Passive immunoglobulin prophylaxis resulted in a reduction in pulmonary virus titer of 300- to 1000-fold, thus demonstrating that pooled monoclonal antibodies directed against major neutralization sites on a major protective viral envelope glycoprotein are effective in vivo against at least two major human viral respiratory tract pathogens, viz., RSV and HPIV3.

It is understood that the examples and embodiments described herein are for illustrative purposes only and that various modifications or changes in light thereof will be suggested to persons skilled in the art.

**TABLE 1**

| Effects of Fc and complement depletion on RSV immunotherapy in cotton rats^{a} | | | | |
|---|---|---|---|---|
| Group no. | No.of animals | Complement depletion, day 2 (CH₅₀ titer⁻¹) | Immunotherapy day 3 (g/kg,i.n.) | Pulmonary virus, day 4 (PFU/g, log₁₀ geometric mean ± SE) |
| 1 | 26 | No (6,700) | No | 5.87 ± 0.04 |
| 2 | 15 | No (6,300) | IgG (0.1) | 3.61 ± 0.24^{b} |
| 3 | 14 | No (NT) | IgG (0.01) | 5.36 ± 0.22 |
| 4 | 17 | No (NT) | IgG (0.001) | 5.91 ± 0.06 |
| 5 | 23 | No (5,600) | F(ab')₂ (0.05) | 3.79 ± 0.18^{b} |
| 6 | 10 | Yes (<50) | No | 5.74 ± 0.07 |
| 7 | 11 | Yes (<50) | IgG (0.1) | 3.07 ± 0.25^{b}^{,}^{c} |
| 8 | 14 | Yes (<50) | F(ab')₂ (0.05) | 4.22 ± 0.14^{b}^{,}^{c} |

| | | | | |
|---|---|---|---|---|
| ^{a} All animals were challenged intranasally on day 0 with 10⁵ PFU of RSV (strain A2). Abbreviations: CH₅₀, 50% hemolytic complement; i.n., intranasally; NT, not tested. | | | | |
| ^{b} P < 0.001, compared with group 1. | | | | |
| ^{c} P < 0.001, compared with group 6. | | | | |

## Claims

1. Use of a pooled suspension of different, purified monoclonal antibodies or F(ab')₂ fragments thereof, directed against conserved epitopes located on major protective antigenic sites of protective antigens of a disease causing virus in the preparation of a small particle aerosol adapted to dispense particles of size equal to or less than about 2 µm for the treatment or prevention of viral respiratory tract disease.

2. Use according to claim 1 wherein the virus is respiratory syncytial virus (RSV).

3. Use according to claim 1 wherein the virus is human parainfluenza virus type 3 (HPIV3).

4. Use according to claim 2 wherein each of the RSV monoclonal antibodies constituting the pooled suspension of monoclonal antibodies, is directed at a unique, highly conserved, protective epitope located on a major protective antigenic site of a major protective viral surface protein and one or more of the MAbs in the pooled suspension are directed at each of the major protective antigenic sites in order to provide effective immunologic prophylactic or therapeutic effect and to prevent the emergence of neutralization-resistant virus mutants during prophylaxis or therapy.

5. Use according to claim 3 wherein each of the HPIV3 monoclonal antibodies constituting the pooled suspension of monoclonal antibodies, is directed at a unique, highly conserved, protective epitope located on a major protective antigenic site of a major protective viral surface protein and one or more of the monoclonal antibodies in the pooled suspension are directed at each of the major protective antigenic sites in order to provide effective immunologic prophylactic or therapeutic effect and to prevent the emergence of neutralization-resistant virus mutants during prophylaxis or therapy.

6. Use according to any of claims 1-5 wherein said monoclonal antibodies are humanized murine-human monoclonal antibodies or human monoclonal antibodies derived from Fab fragments and cloned and expressed in E. coli.

7. Use according to claim 6 wherein F(ab')₂ fragments of said monoclonal antibodies are employed for therapy or prophylaxis.

8. Use according to any one of claims 1-7 wherein the small particle aerosol is adapted to dispense the particles into the lower respiratory tract without intubation.

9. An aerosol which is adapted for administration of small particles of a size equal to or less than 2 µm directly into the lower respiratory tract of a subject at risk from severe viral lower respiratory tract disease, which comprises an effective amount of a pooled suspension of different, purified monoclonal antibodies or F(ab')₂ fragments thereof, directed against conserved epitopes located on major protective antigenic sites of protective antigens of the disease causing virus in association with dispensing means.

10. A pooled suspension of different, purified monoclonal antibodies or F(ab')₂ fragments thereof, directed against conserved epitopes located on major protective antigenic sites of protective antigens of the disease causing virus in the form of an aerosol of particle size equal to or less than 2 µm.

11. The aerosol of claim 9 or suspension of claim 10 wherein the virus is respiratory syncytial virus (RSV).

12. The aerosol of claim 9 or suspension of claim 10 wherein the virus is human parainfluenza virus type 3 (HPIV3).

13. The aerosol or suspension of claim 11 wherein each of the RSV monoclonal antibodies constituting the pooled suspension of monoclonal antibodies, is directed at a unique, highly conserved, protective epitope located on a major protective antigenic site of a major protective viral surface protein and one or more of the MAbs in the pooled suspension are directed at each of the major protective antigenic sites in order to provide effective immunologic, prophylactic or therapeutic effect and to prevent the emergence of neutralization-resistant virus mutants during prophylaxis or therapy.

14. The aerosol or suspension of claim 12 wherein each of the HPIV3 monoclonal antibodies constituting the pooled suspension of monoclonal antibodies, is directed at a unique, highly conserved, protective epitope located on a major protective antigenic site of a major protective viral surface protein and one or more of the monoclonal antibodies in the pooled suspension are directed at each of the major protective antigenic sites in order to provide effective immunologic, prophylactic or therapeutic effect and to prevent the emergence of neutralization-resistant virus mutants during prophylaxis or therapy.

15. The aerosol of claim 9 or suspension of claim 10 wherein said monoclonal antibodies are humanized murine-human monoclonal antibodies or human monoclonal antibodies derived from Fab fragments and cloned and expressed in E. coli.

16. The aerosol or suspension of claim 15 wherein F(ab')₂ fragments of said monoclonal antibodies are employed for therapy or prophylaxis.

17. The aerosol of claim 9 or suspension of claim 10 wherein the small particle size of the aerosol avoids the need to use intubation.

## Patentansprüche

1. Verwendung einer gepoolten Suspension von verschiedenen gereinigten monoklonalen Antikörpern oder F(ab')₂-Fragmenten davon, die gegen konservierte Epitope gerichtet sind, die auf hauptprotektiven antigenen Stellen von protektiven Antigenen eines krankheitauslösenden Virus lokalisiert sind, bei der Herstellung eines Kleinteilchenaerosols, das zur Abgabe von Teilchen einer Größe von gleich oder kleiner als etwa 2 µm geeignet ist, für die Behandlung von oder den Schutz vor einer viralen Atmemwegerkrankung.

2. Verwendung nach Anspruch 1, worin das Virus das respiratorische Synzytialvirus (RSV) ist.

3. Verwendung nach Anspruch 1, worin das Virus Human-Parainfluenzavirus Typ 3 (HPIV3) ist.

4. Verwendung nach Anspruch 2, worin alle monoklonalen RSV-Antikörper, die die gepoolte Suspension monoklonaler Antikörper bilden, auf ein einzigartiges, hoch konserviertes protektives Epitop gerichtet sind, welches auf einer hauptprotektiven antigenen Stelle eines hauptprotektiven viralen Oberflächenproteins lokalisiert ist, und worin einer oder mehrere der MAks in der gepoolten Suspension auf jede der hauptprotektiven antigenen Stellen gerichtet ist, um einen wirksamen immunologischen prophylaktischen oder therapeutischen Effekt zu erzielen und um das Auftreten von neutalisierungsresistenten Virusmutanten während der Prophylaxe oder Therapie zu verhindern.

5. Verwendung nach Anspruch 3, worin alle monoklonalen HPIV3-Antikörper, die die gepoolte Suspension monoklonaler Antikörpern bilden, auf ein einzigartiges, hoch konserviertes protektives Epitop gerichtet sind, das auf einer hauptprotektiven antigenen Stelle eines hauptprotektiven viralen Oberflächenproteins lokalisiert ist, und worin einer oder mehrere der monoklonalen Antikörper in der gepoolten Suspension auf jede der hauptprotektiven antigenen Stellen gerichtet sind, um einen wirksamen immunologischen prophylaktischen oder therapeutischen Effekt zu erzielen und um das Auftreten von neutralisierungsresistenten Virusmutanten während der Prophylaxe oder Therapie zu verhindern.

6. Verwendung nach einem der Ansprüche 1 - 5, worin die monoklonalen Antikörper humanisierte monoklonale Maus-Human-Antikörper oder monoklonale Human-Antikörper sind, die von Fab-Fragmenten stammen und in E. coli kloniert und exprimiert wurden.

7. Verwendung nach Anspruch 6, worin F(ab')₂-Fragmente der monoklonalen Antikörper für Therapie oder Prophylaxe verwendet werden.

8. Verwendung nach einem der Ansprüche 1 - 7, worin das Kleinteilchenaerosol zur Abgabe der Teilchen in den unteren Respirationstrakt ohne Intubation geeignet ist.

9. Aerosol, das geeignet ist zur Verabreichung von kleinen Teilchen mit einer Größe von gleich oder kleiner als 2 µm, direkt in den unteren Respirationstrakt eines Risikopatienten für eine schwere virale Erkrankung des unteren Respirationstraktes, umfassend eine wirksame Menge einer gepoolten Suspension von verschiedenen gereinigten monoklonalen Antikörpern oder F(ab')₂-Fragmenten davon, die gegen konservierte Epitope gerichtet sind, welche auf hauptprotektiven antigenen Stellen von protektiven Antigenen des krankheitsbewirkenden Virus lokalisiert sind, in Verbindung mit Abgabemitteln.

10. Gepoolte Suspension von verschiedenen gereinigten monoklonalen Antikörpern oder F(ab')₂-Fragmenten davon, die gegen konservierte Epitope gerichtet sind, welche auf hauptprotektiven antigenen Stellen von protektiven Antigenen des krankheitsbewirkenden Virus lokalisiert sind, in der Form eines Aerosols mit einer Teilchengröße von gleich oder kleiner als 2 µm.

11. Aerosol nach Anspruch 9 oder Suspension nach Anspruch 10, worin das Virus das respiratorische Synzytialvirus (RSV) ist.

12. Aerosol nach Anspruch 9 oder Suspension nach Anspruch 10, worin das Virus das Human-Parainfluenzavirus Typ 3 (HPIV3) ist.

13. Aerosol oder Suspension nach Anspruch 11, worin alle monoklonalen RSV-Antikörper, welche die gepoolte Suspension monoklonaler Antikörper bilden, auf ein einzigartiges, hoch konserviertes protektives Epitop gerichtet sind, welches auf einer hauptprotektiven antigenen Stelle eines hauptprotektiven viralen Oberflächenproteins lokalisiert ist, und worin einer oder mehrere der MAks in der gepoolten Suspension auf jede der hauptprotektiven antigenen Stellen gerichtet sind, um einen immunologischen prophylaktischen oder therapeutischen Effekt zu erzielen und um das Auftreten von neutalisierungsresistenten Virusmutanten während der Prophylaxe oder Therapie zu verhindern.

14. Aerosol oder Suspension nach Anspruch 12, worin alle monoklonalen HPIV3-Antikörper, welche die gepoolte Suspension monoklonaler Antikörper bilden, auf ein einzigartiges, hoch konserviertes protektives Epitop gerichtet sind, welches auf einer hauptprotektiven antigenen Stelle eines hauptprotektiven viralen Oberflächenproteins lokalisiert ist, und worin einer oder mehrere der monoklonalen Antikörper in der gepoolten Suspension auf jede der hauptprotektiven antigenen Stellen gerichtet sind, um einen immunologischen prophylaktischen oder therapeutischen Effekt zu erzielen und um das Auftreten von neutalisierungsresistenten Virusmutanten während der Prophylaxe oder Therapie zu verhindern.

15. Aerosol nach Anspruch 9 oder Suspension nach Anspruch 10, worin die monoklonalen Antikörper humanisierte monoklonale Maus-Human-Antikörper oder monoklonale Human-Antikörper sind, die von Fab-Fragmenten stammen und in E. coli kloniert und exprimiert wurden.

16. Aerosol oder Suspension nach Anspruch 15, worin F(ab')₂-Fragmente der monoklonalen Antikörper für eine Therapie oder Prophylaxe verwendet werden.

17. Aerosol nach Anspruch 9 oder Suspension nach Anspruch 10, worin die kleine Teilchengröße des Aerosols die Notwendigkeit der Verwendung einer Intubation vermeidet.

## Revendications

1. Utilisation d'une suspension rassemblant différents anticorps monoclonaux purifiés, ou de fragments F(ab') de ceux-ci, dirigés contre des épitopes conservés localisés sur les sites antigéniques protecteurs majeurs des antigènes protecteurs d'un virus pathogène dans la préparation d'un aérosol à petites particules adapté à l'administration de particules d'une taille égale ou inférieure à environ 2 µm pour le traitement ou la prévention d'une maladie virale des voies respiratoires.

2. Utilisation selon la revendication 1, le virus étant le virus respiratoire syncytial (VRS).

3. Utilisation selon la revendication 1, le virus étant le parainfluenzavirus humain de type 3 (HPIV3).

4. Utilisation selon la revendication 2, dans laquelle chacun des anticorps monoclonaux dirigés contre le VRS de la suspension d'un mélange d'anticorps monoclonaux est dirigé contre un épitope protecteur unique fortement conservé localisé sur un site antigénique protecteur majeur d'une protéine protectrice majeure à la surface du virus et un ou plusieurs des Mab de la suspension sont dirigés contre chacun des principaux sites antigéniques protecteurs afin de produire un effet prophylactique ou thérapeutique immunologique efficace et d'éviter l'apparition de virus mutants résistants à la neutralisation au cours de la prophylaxie ou du traitement.

5. Utilisation selon la revendication 3, dans laquelle chacun des anticorps monoclonaux dirigés contre l'HPIV3 de la suspension d'un mélange d'anticorps monoclonaux est dirigé contre un épitope protecteur unique fortement conservé, localisé sur un site antigénique protecteur majeur d'une protéine protectrice majeure à la surface du virus et un ou plusieurs des Mab de la suspension sont dirigés contre chacun des principaux sites antigéniques protecteurs afin de produire un effet prophylactique ou thérapeutique immunologique efficace et d'éviter l'apparition de virus mutants résistants à la neutralisation au cours de la prophylaxie ou du traitement.

6. Utilisation selon l'une quelconque des revendications 1 à 5, dans laquelle lesdits anticorps monoclonaux sont des anticorps monoclonaux murins-humains humanisés ou des anticorps monoclonaux dérivés de fragments Fab et clonés et exprimés dans E. coli.

7. Utilisation selon la revendication 7, dans laquelle des fragments F(ab')₂ desdits anticorps monoclonaux sont utilisés à des fins thérapeutiques ou prophylactiques.

8. Utilisation selon l'une quelconque des revendications 1 à 7, dans laquelle l'aérosol à petites particules est adapté à l'administration de particules dans les voies respiratoires sans intubation.

9. Aérosol adapté à l'administration de petites particules d'une taille égale ou inférieure à 2 µm directement dans les voies respiratoires inférieures d'un sujet à risque en ce qui concerne les maladies virales des voies respiratoires inférieures, comprenant une quantité efficace d'une suspension d'un mélange de différents anticorps monoclonaux purifiés ou de fragments F(ab')₂ de ceux-ci, dirigés contre des épitopes conservés localisés au niveau de sites antigéniques protecteurs majeurs des antigénes protecteurs du virus responsable de la maladie en association avec des moyens de dispersion.

10. Suspension d'un mélange de divers anticorps monoclonaux purifiés ou de fragments F(ab')₂ de ceux-ci, dirigés contre des épitopes conservés localisés au niveau de sites antigéniques protecteurs majeurs des antigènes protecteurs du virus responsable de la maladie sous la forme d'un aérosol dont la taille des particules est égale ou inférieure à 2 µm..

11. Aérosol de la revendication 9 ou suspension de la revendication 10, le virus étant le virus respiratoire syncytial (VRS).

12. Aérosol de la revendication 9 ou suspension de la revendication 10, le virus étant le parainfluenzavirus humain de type 3 (HPIV3).

13. Aérosol ou suspension de la revendication 11, dans lequel chacun des anticorps monoclonaux dirigés contre le VRS de la suspension d'un mélange d'anticorps monoclonaux est dirigé contre un épitope protecteur unique fortement conservé, localisé sur un site antigénique protecteur majeur d'une protéine protectrice majeure à la surface du virus et un ou plusieurs des Mab de la suspension sont dirigés contre chacun des principaux sites antigéniques protecteurs afin de produire un effet prophylactique ou thérapeutique immunologique efficace et d'éviter l'apparition de virus mutants résistants à la neutralisation au cours de la prophylaxie ou du traitement.

14. Aérosol ou suspension de la revendication 12, dans lequel chacun des anticorps monoclonaux dirigés contre l'HPIV3 de la suspension d'un mélange d'anticorps monoclonaux est dirigé contre un épitope protecteur unique fortement conservé, localisé sur un site antigénique protecteur majeur d'une protéine protectrice majeure à la surface du virus et un ou plusieurs des Mab de la suspension sont dirigés contre chacun des principaux sites antigéniques protecteurs afin de produire un effet prophylactique ou thérapeutique immunologique efficace et d'éviter l'apparition de virus mutants résistants à la neutralisation au cours de la prophylaxie ou du traitement.

15. Aérosol de la revendication 9 ou suspension de la revendication 10, dans lequel lesdits anticorps monoclonaux sont des anticorps monoclonaux murins-humains humanisés ou des anticorps monoclonaux dérivés de fragments Fab et clonés et exprimés dans E. coli.

16. Aérosol ou suspension de la revendication 15, dans lequel les fragments F(ab')₂ desdits anticorps monoclonaux sont utilisés à des fins thérapeutiques ou prophylactiques.

17. Aérosol de la revendication 9 ou suspension de la revendication 10 dans lequel la petite taille des particules évite la nécessité de l'utilisation d'une intubation.
